(19) Europäisches Patentamt
European Patent Office
Office européen des brevets

(11) EP 1 554 404 B1

(12) **EUROPEAN PATENT SPECIFICATION**

(45) Date of publication and mention of the grant of the patent:
**22.04.2009 Bulletin 2009/17**

(21) Application number: **03758305.1**

(22) Date of filing: **10.10.2003**

(51) Int Cl.:
***C12Q 1/68*** *(2006.01)*

(86) International application number:
**PCT/GB2003/004412**

(87) International publication number:
**WO 2004/033726 (22.04.2004 Gazette 2004/17)**

# (54) DETECTION SYSTEM

NACHWEISSYSTEM

SYSTEME DE DETECTION

(84) Designated Contracting States:
**AT BE BG CH CY CZ DE DK EE ES FI FR GB GR HU IE IT LI LU MC NL PT RO SE SI SK TR**

(30) Priority: **10.10.2002 GB 0223563**

(43) Date of publication of application:
**20.07.2005 Bulletin 2005/29**

(73) Proprietor: **THE SECRETARY OF STATE FOR DEFENCE**
**Salisbury**
**Wiltshire SP4 0JQ (GB)**

(72) Inventors:
- **LEE, Martin, Alan**
  **Salisbury,**
  **Wiltshire SP4 0JQ (GB)**
- **BASCHE, Mark**
  **Salisbury**
  **Wiltshire SP4 0JQ (GB)**
- **BROWN, Tom**
  **Southampton, S017 1BJ (GB)**

(74) Representative: **Greaves, Carol Pauline et al**
**Greaves Brewster LLP**
**Indigo House**
**Cheddar Business Park**
**Wedmore Road**
**Cheddar, Somerset BS27 3EB (GB)**

(56) References cited:
**EP-A- 0 699 768**
**EP-A- 0 872 562**
**WO-A-99/28500**
**WO-A-02/097132**
**US-A- 5 208 323**
**US-A- 5 858 397**
**US-A1- 2002 106 682**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 554 404 B1

Printed by Jouve, 75001 PARIS (FR)

## Description

The present invention provides a method for detecting a target polynucleotide in a sample, for example by quantitatively monitoring an amplification reaction, as well as to probes and kits for use in these methods. The method is particularly suitable for the detection of polymorphisms or allelic variation and so may be used in diagnostic methods

Known fluorescence polymerase chain reaction (PCR) monitoring techniques include both strand specific and generic DNA intercalator techniques that can be used on a few second-generation PCR thermal cycling devices. These reactions are carried out homogeneously in a closed tube format on thermal cyclers (see EP-A-872562). Reactions are monitored using a fluorimeter. The precise form of the assays varies but often relies on fluorescence energy transfer or FET between two fluorescent moieties within the system in order to generate a signal indicative of the presence of the product of amplification. EP-A-0699768 describes methods for in situ quenching of fluorescently labelled olignonucleotide probes.

WO 99/28500 describes a very successful assay for detecting the presence of a target nucleic acid sequence in a sample. In this method, a DNA duplex binding agent and a probe specific for said target sequence, is added to the sample. The probe comprises a reactive molecule able to absorb fluorescence from or donate fluorescent energy to said DNA duplex binding agent. This mixture is then subjected to an amplification reaction in which target nucleic acid is amplified, and conditions are induced either during or after the amplification process in which the probe hybridises to the target sequence. Fluorescence from said sample is monitored.

As the probe hybridises to the target sequence, DNA duplex binding agent such as an intercalating dye is trapped between the strands. In general, this would increase the fluorescence at the wavelength associated with the dye. However, where the reactive molecule is able to absorb fluorescence from the dye (i.e. it is an acceptor molecule), it accepts emission energy from the dye by means of FET, especially FRET, and so it emits fluorescence at its characteristic wavelength. Increase in fluorescence from the acceptor molecule, which is of a different wavelength to that of the dye, will indicate binding of the probe in duplex form.

Similarly, where the reactive molecule is able to donate fluorescence to the dye (i.e. it is a donor molecule), the emission from the donor molecule is reduced as a result of FRET and this reduction may be detected. Fluorescence of the dye is increased more than would be expected under these circumstances.

The signal from the reactive molecule on the probe is a strand specific signal, indicative of the presence of target within the sample. Thus the signal changes in fluorescence from the reactive molecule, which are indicative of the formation or destabilisation of duplexes involving the probe, are preferably monitored.

DNA duplex binding agents, which may be used in the process, are any entity which adheres or associates itself with DNA in duplex form and which is capable of acting as an energy donor or acceptor. Particular examples are intercalating dyes as are well known in the art.

The use of a DNA duplex binding agent such as an intercalating dye and a probe which is singly labelled is advantageous in that these components are much more economical than other assays in which doubly labelled probes are required. By using only one probe, the length of known sequence necessary to form the basis of the probe can be relatively short and therefore the method can be used, even in difficult diagnostic situations.

The DNA duplex binding agent used in the assay is typically an intercalating dye, for example SYBRGreen such as SYBRGreen I, SYBRGold, ethidium bromide and YOPRO-1, which are themselves fluorescent.

In order for FET, such as FRET, to occur between the reactive molecule and the dye, the fluorescent emission of the donor (which may either be the intercalating dye or the reactive molecule on the probe) must be of a shorter wavelength than the acceptor (i.e. the other of the dye or the reactive molecule). The fluorescent signals produced by the molecules used as donor and/or acceptor can be represented as peaks within the visible spectrum.

Generally, there will be at least some overlap in the wavelengths of the emission. Even where the signals are sharp peaks, there will be some "leakage" of signal from fluorescent molecules so that it is generally necessary to resolve the strand specific peak produced by the probe from the DNA duplex binding agent signal. This can be done, for example by determining empirically the relationship between the spectra of the donor and acceptor and using this relationship to normalise the signals from the donor and acceptor.

The applicants have found an improved way of operating an assay of this type.

The present invention provides a method for detecting the presence of a target nucleic acid sequence in a sample, said method comprising:

(a) adding to a sample suspected of containing said target nucleic acid sequence, a fluorescently labelled probe specific for said target sequence, and a DNA duplex binding agent which can absorb fluorescent energy from the fluorescent label on the probe whose emissions are not detectable in the context of the method,
(b)subjecting the thus formed mixture to an amplification reaction in which target nucleic acid is amplified,
(c) subjecting said sample to conditions under which the said probe hybridises to the target sequence, and
(d) monitoring fluorescence from said sample; wherein the probe is released intact from the target sequence.

The expression "visible light" used herein refers to radiation in the visible region of the spectrum, i.e. at wavelengths in the range of 390nm to 750nm.

By using a DNA duplex binding agent as described above, the problem with it supplying a signal that may overlap with that of the probe is avoided. Thus the need to resolve the signals from the probe from the signal from the DNA duplex binding agent is eliminated, and a broader bandwidth over which meaningful signal can be measured is available. This means that the apparatus, or at least the computational requirements placed upon the apparatus can be simplified.

The assay may therefore be carried out on a broader range of instruments.

Alternatively, any areas of free bandwidth in the visible spectrum may be exploited by incorporating additional probes, which include different which fluoresce at different wavelengths so that more that one target may be monitored at the same time. This may be particularly useful in the case of multiplex PCR reactions.

In one embodiment, the DNA duplex binding agent, which is used, is a compound which bind to a DNA duplex, but does not emit radiation in the visible portion of the spectrum.

The DNA duplex binding agent may be an intercalating agent, a minor groove binder, a compound which binds to DNA major groove, or a compound which binds or stack onto an end base of a probe, as well as combinations therof. In particular embodiments, it will comprise an intercalating agent or a minor groove binder. It may emit radiation for examples at wavelengths outside the visible range of the spectrum, for example in the infrared range. However, such emissions would not be detectable in the context of the method of the invention, and so effectively the DNA duplex binding agent acts only as a "dark quencher".

Such compounds are frequently more economical than conventional fluorescent intercalating dyes, making the process of the invention more cost effective.

Examples of suitable DNA binding agents, which may be used in this way, include DNA binding agents that have conjugated aromatic ring systems. Rings may be aryl rings, such as phenyl, napthyl or anthracene rings, or aromatic heterocyclic rings, for example containing up to 20 atoms, up to five of which are heteroatoms such as oxygen, sulphur and nitrogen. Examples of such systems include anthracyclins or anthraquinones. These may be substituted to provide the appropriate DNA binding properties.

In particular, compounds comprise an optionally substituted anthraquinone of structure (I)

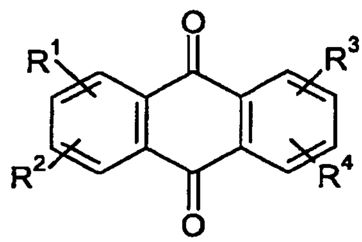

(I)

where $R^1$, $R^2$, $R^3$ and $R^4$ are independently selected from hydrogen, a functional group, or a hydrocarbyl group optionally substituted by for example functional groups, or $R^1$ and $R^2$ or $R^3$ and $R^4$ are optionally joined together to form a ring which optionally contains heteroatoms, and/or is optionally substituted by a functional group or a hydrocarbyl group.

As used herein, the term "functional group" refers to a reactive group, which suitably contains a heteroatom. Examples of functional groups include halo, cyano, nitro, oxo, $-OC(O)R^a$, $-OR^a$, $-C(O)OR^a$, $S(O)_tR^a$, $NR^bR^c$, $OC(O)NR^bR^c$, $C(O)NR^bR^c$, $OC(O)NR^bR^c$, $-NR^aCONR^bR^c$, $-C{=}NOR^a$, $-N{=}CR^bR^c$, $S(O)_tNR^bR^c$, $C(S)_nR^a$, $C(S)OR^a$, $C(S)NR^bR^c$ or $-NR^bS(O)_tR^a$ where $R^a$, $R^b$ and $R^c$ are independently selected from hydrogen or optionally substituted hydrocarbyl, or $R^b$ and $R^c$ together form an optionally substituted ring which optionally contains further heteroatoms such as $S(O)_s$, oxygen and nitrogen, n' is an integer of 1 or 2, s is 0, 1 or 2, t is 0 or an integer of 1-3.

Suitable optional substituents for hydrocarbyl groups $R^a$, $R^b$ and $R^c$ may also be functional groups.

As used herein the term "hydrocarbyl" refers to organic groups comprising carbon and hydrogen atoms such as alkyl, alkenyl, alkynyl, cycloalkyl, aryl or aralkyl. The term "alkyl" refers to straight or branched chain alkyl group, suitably containing up to 20, more suitably up to 10 and preferably up to 6 carbon atoms. The term "alkenyl" or "alkynyl" refers to unsaturated straight or branched chains, having from 2 to 10 carbon atoms. The term "cycloalkyl" refers to alkyl groups which have at least 3 carbon atoms, and which are cyclic in structure. The term "aryl" refers to aromatic rings such as phenyl and naphthyl. The term aralkyl refers to alkyl groups substituted by aryl groups such as benzyl.

Particular examples of substituents for $R^1$, $R^2$, $R^3$ and $R^4$ are hydroxy groups so as to give rise to keto-enol tautomerism.

Preferably the compound contains one or more heteroatoms, to give a charge which will assist in binding to

DNA. The heteroatoms, such as oxygen, nitrogen or sulphur, may be included in the substituent side chains. In particular embodiments, the compounds of formula (I) include at least one nitrogen atom within the substituents $R^1$, $R^2$, $R^3$ and $R^4$.

Examples of such compounds may be found in the pharmaceutical fields, and in particular in anticancer or antibiotic applications, as a result of the DNA binding functionality. For examples, compounds which may have the properties which make them suitable for use as DNA binding agents in the assay of the present invention include US Patent No. 4197249, US Patent No 3183157, US Patent No 4012284 and US Patent No.3997662.

Other specific compounds are compounds of formula (IA) and are described in US Patent No 513327. These compounds are of formula (I) as described above but in that case, $R^1$, $R^2$, $R^3$ and $R^4$ are independently selected from hydrogen, X, NH-ANHR and NH-A-N (O) R' R" where X is hydroxy, halo, amino, $C_{1-4}$alkoxy or $C_{2-8}$alkanoyloxy, A is a $C_{2-4}$alkylene group with a chain length between NH and NHR or N(O)R'R" of at least 2 carbon atoms and R, R' and R" are each independently selected from $C_{1-4}$alkyl and $C_{2-4}$hydroxyalkyl and $C_{2-4}$dihydroxyalkyl, provided that a carbon atom attached to a nitrogen atom does not carry a hydroxy group and that no carbon atom is substituted by two hydroxy groups; or R' and R" together are a $C_{2-6}$alkylene group which, with the nitrogen atom to which R' and R" are attached for a heterocyclic ring having 3 to 7 atoms, with the proviso that at least one of $R^1$, $R^2$, $R^3$ and $R^4$ is a group NH-A-N (O)R'R". Particular examples are described in US Patent No. 5132,327, the content of which is included herein by reference.

Compounds which may be suitable for use as DNA duplex binding agents in the invention may be tested to see whether or not they absorb fluorescent energy for example, from a particular or from a range of labels using conventional methods. In particular, they may be included in a PCR reaction with a fluorescent agent, which may be a labelled probe or even a fluorescent intercalating agent such as Sybr Green or Sybr Gold, to test the quenching properties, and also to ensure that they do not impede the progress of the amplification reaction itself. A suitable protocol for carrying out this testing is set out in Example 3 hereinafter.

Particular examples are mitoxantrone (1,4-dihydroxy 5,8-bis[[2-[(2-hydroxyethyl)amino]ethyl]amino]-9,10-anthracenedione) or it salt such as the hydrochloride or dihydrochloride salt, nogalamycin (2R-(2α,3β,4α,6α,11β,13α,14α)]-11-[6-deoxy-3-C-mehtyl-2,3,4-tri-O-methyl-α-L-mannopyranosyl)oxy]-4-(dimethylamino)-3,4,5,6,9,11,12,13,14,16-decahydro-3,5,8,10,13-pentahydroxy-6,13-dimethyl-9,16-dioxo-2,6-epoxy-2H-naphthaceno[1,2-b]oxocin-14-carboxylic acid methyl ester) or daunomycin (8S,-cis)-8-acetyl-10-[3-amino-2,3,6-trideoxy-α-L-lyxo-hexopyranosyl)oxy]-7,8,9,10-tetrahydro-6,8,11-trihydroxy-1-methoxy-5,12-naphthacendione).

Other specific examples include compounds described in US Patent No. 5132327 (equivalent to EP-A-0450021), and in particular the cell permeant DNA compound available from Biostatus under the trade name, 'Draq5', and the N-oxide derivative of this available under the trade name 'Apoptrak'.

A particular group of DNA duplex binding agents for use in the invention are mitroxanone, daunomycin, Draq5™ and Apoptrak™.

In a particular embodiment, the DNA duplex binding agent is mitoxantrone.

Alternatively or additionally a quenching moiety such as 4-(4-dimethylaminophenylazo) benzoic acid (DABCYL) may be attached and preferably covalently bound, to a known DNA binding, intercalating or minor or major groove binding agent. In this case, the DNA binding agent may have some degree of fluorescence provided that this is entirely quenched by the quenching moiety. These compounds have the effect of stabilising the duplex. This is advantageous in two respects. Firstly it improves the binding of the probe to the target, reducing the time taken to change temperatures during the amplification, and so allowing the reaction to be carried out faster. Secondly it allows the use of shorter nucleic acid sequences for primers and probes. This is generally useful where for example melt point analysis is being carried out, since the shorter the probe, the more significant will be the difference between melting points caused by mismatches. It may be particularly useful in for example AT rich targets where long primers and probes can reduce the specificity of the reaction because of the low temperatures that may be required for probe and primer annealing.

The quenching effects of the DNA duplex binding agent may be felt to some extent by the probe when in single stranded form. However, the quenching will be significantly and distinguishably more pronounced in the case of duplex DNA. Generally any free label present in the system will not be subject to quenching by the DNA duplex binding agent, since no association forms between them.

The amount of DNA duplex binding agent which is added to the reaction mixture is suitably sufficient to cause measurable quenching of the signal from the fluorescent label, but not sufficient to inhibit amplification. The range of concentrations which will achieve this vary depending upon the precise DNA duplex binding agent used, and can be determined by routine methods as illustrated hereinafter. For DNA duplex binding agents such as mitoxantrone or daunomycin, concentrations of the order of 1μM to 100μM and suitably about 10μM-25μM would be employed. For Draq5, concentrations in the range of from 1μM to 100μM, and preferably about 50μM, are effective in quenching a single labelled fluorescein probe. Higher concentrations, for instance of 1mM of Apoptrak™ may be required to satisfactorily quench a fluorescein labelled probe.

The method of the invention is extremely versatile in its applications. The method can be used to generate both quantitative and qualitative data regarding the target nucleic acid sequence in the sample, as discussed in more detail

hereinafter. In particular, not only does the invention provide for quantitative amplification, but also it can be used, additionally or alternatively, to obtain characterising data such as duplex destabilisation temperatures or melting points.

In the method of the invention, the sample may be subjected to conditions under which the probe hybridises to the samples before, during or after the amplification reaction. The process therefore allows the detection to be effected in a homogenous manner, in that the amplification and monitoring can be carried out in a single container with all reagents added initially. No subsequent reagent addition steps are required. Neither is there any need to effect the method in the presence of solid supports (although this is an option).

The probe may comprise a nucleic acid molecule such as DNA or RNA, which will hybridise to the target nucleic acid sequence when the latter is in single stranded form. In this instance, step (c) will involve the use of conditions which render the target nucleic acid single stranded.

Probe may either be free in solution or immobilised on a solid support, for example to the surface of a bead such as a magnetic bead, useful in separating products, or the surface of a detector device, such as the waveguide of a surface plasmon resonance detector. The selection will depend upon the nature of the particular assay being looked at and the particular detection means being employed.

In particular, the amplification reaction used will involve a step of subjecting the sample to conditions under which any of the target nucleic acid sequence present in the sample becomes single stranded. Such amplification reactions include the polymerase chain reaction (PCR) or the ligase chain reaction (LCR), but is preferably a PCR reaction.

It is possible then for the probe to hybridise during the course of the amplification reaction provided appropriate hybridisation conditions are encountered.

In a preferred embodiment, the probe may be designed such that these conditions are met during each cycle of the amplification reaction. Thus at some point during each cycle of the amplification reaction, the probe will hybridise to the target sequence, and whereupon the fluorescent signal will be quenched as a result of its close proximity to the DNA duplex binding agent trapped between the probe and the target sequence. As the amplification proceeds, the probe will be separated or melted from the target sequence and so the signal generated by it will be restored. Hence in each cycle of the amplification, a fluorescence peak from the fluorescent label at the point at which the probe is annealed is generated. The intensity of the peak will decrease as the amplification proceeds because more target sequence becomes available for binding to the probe.

By monitoring the fluorescence of the fluorescent label in the sample during each cycle, the progress of the amplification reaction can be monitored in various ways. For example, the data provided by melting peaks can be analysed, for example by calculating the area under the melting peaks and this data plotted against the number of cycles.

Fluorescence is suitably monitored using a known fluorimeter. The signals from these, for instance in the form of photomultiplier current, are sent to a data processor board and converted into a spectrum associated with each sample tube. Multiple tubes, for example 96 tubes, can be assessed at the same time. Data may be collected in this way at frequent intervals, for example once every 10ms, throughout the reaction.

This data provides the opportunity to quantitate the amount of target nucleic acid present in the sample.

In addition, the kinetics of probe hybridisation will allow the determination, in absolute terms, of the target sequence concentration. Changes in fluorescence from the sample can allow the rate of hybridisation of the probe to the sample to be calculated. An increase in the rate of hybridisation will relate to the amount of target sequence present in the sample.

As the concentration of the target sequence increases as the amplification reaction proceeds, hybridisation of the probe will occur more rapidly. Thus this parameter also can be used as a basis for quantification. This mode of data processing useful in that it is not reliant on signal intensity to provide the information.

Suitable fluorescent labels are rhodamine dyes or other dyes such as Cy5, Cy3, Cy5.5, fluorescein or derivatives thereof. Particular derivatives are carboxyfluorescein compounds sold under the trade name FAM, such as 5-carboxyfluorescein, 6-carboxyfluorescein, or their succinimidyl esters.

The selection of the fluorescent label will usually be related to the choice of absorbing agent. Clearly the label should be one whose fluorescence should be in a range which can be absorb by the intercalating agent.

Mitoxantrone, daunomycin, Draq5 and Apoptrak are particularly good quenchers of fluorescein and its derivatives, and in particular FAM compounds.

The labels may be attached to the probe in a conventional manner. The position of the fluorescent label along the probe is immaterial although it general, they will be positioned at an end region of the probe.

Preferably they are positioned at the 3' end of the probe, as they will then act as a steric or chemical blocking agent, to prevent extension of the probe by the polymerase during the amplification. This may avoid the need to take other measures, such as phosphorylation, in order to block the 3' end of the probe during the amplification reaction.

It is not necessary in this assay for the probe to be consumed as signal production is achieved without dissociating the probe.

In order to achieve a fully reversible signal which is directly related to the amount of amplification product present at each stage of the reaction, and/or where speed of reaction is of the greatest importance, for example in rapid

PCR, it is preferable that the probe is designed such that it is released intact from the target sequence. This may be, for example, during the extension phase of the amplification reaction. However, since the signal is not dependent upon probe hydrolysis, the probe may be designed to hybridise and melt from the target sequence at any stage during the amplification cycle. For example probes which hybridise most strongly at a stage other than the extension phase of the cycle will ensure that interference with the amplification reaction is minimised.

Where probes which bind strongly at or below the extension temperature are used, their release intact from the target sequence can be achieved by using a 5'-3' exonuclease lacking enzyme such as Stoffle fragment of Taq or Pwo, as the polymerase in the amplification reaction.

The probe may then take part again in the reaction, and so represents an economical application of probe.

The data generated in this way using probes which reversibly hybridise to the target and are not hydrolysed, can be interpreted in various ways. In its simplest form, a decrease in fluorescence of the fluorescent label at the probe annealing temperature in the course of or at the end of the amplification reaction is indicative of an increase in the amount of the target sequence present, suggestive of the fact that the amplification reaction has proceeded and therefore the target sequence was in fact present in the sample.

However, as outlined above, quantification is also possible by monitoring the amplification reaction throughout.

Finally, it is possible to obtain characterisation data and in particular melting point analysis, either as an end point measure or throughout, in order to obtain information about the sequence as will be discussed further below.

Thus, a preferred embodiment of the invention comprises a method for detecting nucleic acid amplification comprising:

> performing nucleic acid amplification on a target polynucleotide in the presence of (a) a nucleic acid polymerase (b) at least one primer capable of hybridising to said target polynucleotide, (c) an oligonucleotide probe which is capable of binding to said target polynucleotide sequence and which contains a fluorescent label and (d) a DNA duplex binding agent which is capable of absorbing fluorescent energy from the said fluorescent label, and whose emissions are not detectable in the context of the method; and monitoring changes in fluorescence during the amplification reaction.

The amplification is suitably carried out using a pair of primers which are designed such that only the target nucleotide sequence within a DNA strand is amplified as is well understood in the art. The nucleic acid polymerase is suitably a thermostable polymerase such as Taq polymerase.

Suitable conditions under which the amplification reaction can be carried out are well known in the art. The optimum conditions may be variable in each case depending upon the particular amplicon involved, the nature of the primers used and the enzymes employed. The optimum conditions may be determined in each case by the skilled person. Typical denaturation temperatures are of the order of 95°C, typical annealing temperatures are of the order of 55°C and extension temperatures are of the order of 72°C.

Suitably, the fluorescence is monitored throughout the amplification process, and preferably, at least at the same point during each amplification cycle. In particular, fluorescence needs to be monitored at the temperature at which the probe anneals to the target. For instance, this may be at a temperature of about 60°C.

As more target is formed, more probe becomes annealed to it, and is quenched as a result of it being brought into close proximity to the DNA duplex binding agent. This reduction in fluorescence indicates the progress of the amplification.

The polymerase such as TAQ™ polymerase present in the sample will have the effect of removing the probe from the target.

This effect occurs at a low level, at the sub-optimal temperature for the polymerase, such as the probe annealing temperature. Hence at this temperature, these two reactions, the binding of the probe at its annealing temperature and the effect of the polymerase to remove the probe from the target, will compete. Generally, the former reaction will dominate for a significant number of reaction cycles, allowing the amplification reaction to be monitored. Ultimately however, a rise in fluorescence may be observed, when the balance shifts and the effect of the polymerase becomes more dominant. Hence the results can reveal a "hook" effect, caused by the rise in fluorescence at the end of the amplification reaction.

The data obtained using the method of the invention, can be processed to monitor the progress of the amplification reaction, and may therefore be used to quantify the amount of target present in the sample.

In order to interpret the data obtained, it may be necessary to make certain adjustments. For instance, in a conventional PCR monitoring reaction such as that described in WO 99/28500, the PCR reaction will lead to an exponential rise in fluorescence, and so baseline adjustments for background fluorescence will need to be derived from the lowest values obtained.

In contrast, in the method of the present application, the progress of a PCR reaction will lead to an exponential fall in fluorescence as progressively more of the labelled probe is quenched by the DNA duplex binding agent. Hence

baseline adjustment needs to be based upon the highest levels of fluorescence achieved.

This is suitably done by taking the data from a sample reaction reaction and applying the following equations to every datapoint:

$$y=1/x$$

$$z=y-MIN$$

where x is the datapoint from the PCR machine, such as a LightCyler, Z is the baseline adjusted datapoint and MIN is the minimum value for y over the entire dataset. A plot of Z vs cycle number will allow appropriate baseline adjustments to be calculated.

The method can be used in hybridisation assays for determining characteristics of particular sequences.

Thus in a further aspect, the invention provides a method for determining a characteristic of a sequence, said method comprising;

a) adding to a sample suspected of containing said sequence, a fluorescently labelled probe specific for said target sequence and a DNA duplex binding agent able to absorb fluorescence from a fluorescent label on the probe whose emissions are not detectable in the context of the method,
(b) subjecting said sample to conditions under which the said probe hybridises to the target sequence and thereafter is released intact from the target sequence,
(c) monitoring fluorescence from said sample and determining a particular reaction condition, characteristic of said sequence, at which fluorescence changes as a result of the release intact of the probe from the target nucleic acid sequence.

Suitable reaction conditions include temperature, electrochemical, or the response to the presence of particular enzymes or chemicals. By monitoring changes in fluorescence as these properties are varied, information characteristic of the precise nature of the sequence can be determined. For example, in the case of temperature, the temperature at which the probe separates or "melts" from the target sequence can be determined. This can be extremely useful in for example, to detect and if desired also to quantitate, polymorphisms in sequences including allelic variation in genetic diagnosis. By "polymorphism" is included transitions, transversions, insertions, deletions or inversions which may occur in sequences, particularly in nature.

The hysteresis of melting of the probe will be different if the target sequence varies by only one base pair. Thus where a sample contains only a single allelic variant, the temperature of melting of the probe will beta particular value which will be different from that found in a sample which contains only another allelic variant. A sample containing both allelic variants which show two melting points corresponding to each of the allelic variants.

Similar considerations apply with respect to electrochemical properties, or in the presence of certain enzymes or chemicals. The probe may be immobilised on a solid surface across which an electrochemical potential may be applied. Target sequence will bind to or be repulsed from the probe at particular electrochemical values depending upon the precise nature of the sequence.

This embodiment can be effected in conjunction with amplification reactions such as the PCR reaction mentioned above, or it may be employed individually.

Kits for use in the method of the invention may be provided. These kits will contain a DNA duplex binding agent as described above, for example one which is able to absorb fluorescent energy from a fluorescent label which may be found on a probe, but which does not emit light in the visible range of the spectrum. The kit will additionally include a probe for a target sequence which is fluorescently labelled, an amplification primer as well as a 5'-3'exonuclease lacking polymerase enzyme. Other potential components of the kit include reagents used in amplification reactions such buffers and adjuncts known to improve the PCR process such as the "hotstart" reagents such as antiTaq antibody, or pyrophosphate and a pyrophosphatase, as described in copending International Patent Application PCT/GB02/01861.

The kits may include all the reagents together in a single container, or some may be in separate containers for mixing on site.

The invention will now be particularly described by way of example with reference to the accompanying diagrammatic drawings in which:

Figure 1 shows diagrammatically the interactions which occur using the method of the invention;

Figure 2 illustrates stages during an amplification reaction in accordance with the invention;

Figures 3 is a graph showing the results of an amplification reaction in accordance with the invention, plotting the inverse of fluorescence occurring at the end of the annealing step, against cycle number, and illustrating the effect of 1:100 of 0.0193M mitoxantrone on three 10 fold dilutions of human placental DNA;

Figure 4 is a graph showing the quenching effect of a 10 fold dilution series of the neat (0.0193M) mitoxantrone on the CTW19 probe;

Figure 5 is a graph showing the quenching effect of a 10 fold dilution series of the neat (5mM) daunomcyin on the CTW19 probe, and

Figure 6 is a graph illustrating the effect on fluorescence of inclusion of a dark quencher at various concentrations on a PCR reaction carried out in the presence of a FAM labelled probe.

An element of the method of the invention is a probe (1) which carries a fluorescent label (2), preferably at the 3' end. This probe, which specifically binds the target sequence, is added to the sample suspected of containing the target sequence together with a DNA duplex binding agent (3).

When the probe (1) is free in solution, the fluorescent label (2) will fluoresce. Some DNA duplex binding agent may become associated with the probe which may quench the signal slightly, but the level of quenching is low (Figure 1A). However, when the probe (1) hybridises with a single stranded target sequence (4) to form a duplex as illustrated in Figure 1B, DNA duplex binding agent (3) becomes associated with the duplex and is therefore brought into close proximity to the fluorescent label. Fluorescent energy from the label passes to the DNA duplex binding agent (3), and so the fluorescence from the sample is reduced or quenched. Decrease in the fluorescence of the label will thus be indicative of hybridisation of the probe to the target sequence.

Thus by measuring the decrease in fluorescence of the label, for example as the temperature decreases, the point at which hybridisation occur can be detected. Similarly, an increase in label fluorescence will occur as the temperature increases at the temperature at which the probe (1) melts from the target sequence (4), as the label is no longer affected by the DNA duplex binding agent.

The melt temperature will vary depending upon the hybridisation characteristics of the probe and the target sequence. For example, a probe, which is completely complementary to a target sequence, will melt at a different temperature to a probe that hybridises with the target sequence but contains one or more mismatches.

Figure 2 illustrates how the method of the invention can be employed in amplification reactions such as the PCR reaction. Probe (1) will hybridise to single stranded DNA in conjunction with the DNA duplex binding agent (3) and thus the label signal will be quenched (Figure 2A). In the illustrated embodiment this occurs during the annealing phase of the cycle during which the primer (5) anneals. As the amount of target sequence increases as a result of the amplification, the signal generated during the annealing phase by the label will decrease as a result of increased quenching by the formation of more duplexes which incorporate the probe and also the DNA duplex binding agent.

During the extension phase, the probe is removed from the target sequence because the DNA polymerase displaces it. At this point, the label signal increases because the probe moves away from the DNA duplex binding agent (Figure 2B).

By monitoring the fluorescence from the label, the progress of the amplification reaction can be followed and the quantity of target sequence present in the original sample can be determined.

Example 1

PCR amplification reaction

The method of the invention was tested using the Carl Wittwer assay for the human beta Globin gene. In each case, the following experimental protocol was followed.

First of all, 10mls of a 2x Master mix formulation was prepared comprising the following components:

| | |
|---|---|
| 2x Master Mix Formulation: | 2000μl Tris pH 8.8 at 500mM |
| | 2000μl dUTP Nucleotides at 2mM |
| | 250μl B.S.A at 20mg/ml |
| | 1600μl Glycerol |
| | 200μl Uracil-N-Glycosylase at 1 unit/μl |
| | 160μl Taq Polymerase at 5 units/μl |

3190μl HPLC Grade Water
600μl Magnesium Chloride solution at 0.1M

A PCR mix formulation, suitable for conducting the Carl Wittwer assay, was then prepared and comprised the following components:

PCR Mix Formulation: 50μl of 2x Master mix at 3mM $Mg^{2+}$
10μl of Forward Primer (PC03) at 10μM
10μM of Reverse Primer (PCO4) at 10μM
10μl of Probe (CTW19) at 2μM
5μl of HPLC Grade Water
5μl of Mitoxantrone at 10μM concs

Primer sequence (PCO3) ACA CAA CTG TGT TCA CTA GC
Primer sequence (PC04): CAA CTT CAT CCA CGT TCA CC
CTW19: CAA ACA GAC ACC ATG GTG CAC CTG ACT CCT GAG GAT (3' fluorescein)

This PCR mix formulation constituted 90μl in total. The mix was then vortexed thoroughly and split into 2 x 45μl. To one of these was added 5μl of HPLC grade water to act as No Template Control's (NTC's) and to the other 5μl of human placental DNA (Various Concentrations) was added to act as the Positives. These 2 x 50μl were then further split into 4 x μl and pipetted into Lightcycler capillaries to create NTC's in duplicate and +'s in duplicate

The above mix would be made for each value of the variable(s) being tested in each experiment.

The capillaries were then spun down and run on the Roche Lightcycler on the following cycle programme:

<u>Carry over prevention x 1</u>

50°C for 60 seconds
95°C for 15 seconds

<u>Cycle x 50</u>

95°C. for 5 seconds
60°C for 5 seconds. Fluorescence collected at this step in F1 channel (530nm)
74°C for 5 seconds

<u>Melt analysis x 1</u>

50°C for 15 seconds
Slow ramp to 95°C at 0.1°C/second. Fluorescence collected throughout this step in F1 channel (530nm)

A typical result is shown in Figure 3.

Figure 3 illustrates that for a 10 fold dilution series, a distinguishable signal, above that of background. A tenfold dilution of target template in an optimium PCR, where the amplification would be such that exponential amplification occurs, would result in increase in the number of amplicons by a factor of 2 every cycle. A probe system that is used to detect the concentration of amplicons, and by inference the initial amount of target, should generate signals that will rise above background at an arbitrary cycle values that are ~3.31 cycles apart for each 10 fold dilution within the functional range of the PCR. This is clearly shown in Figure 3.

<u>Example 2</u>

<u>Determination of Optimum concentration of DNA duplex binding agents</u>

The PCR reaction as described in Example 1 was repeated using various concentrations of DNA duplex binding agents, mitoxantrone and daunomcyin. The results are shown in Figures 4 and 5 respectively. It is clear from these Figures that clear signals representing the amplification reaction appeared where the starting mitoxantrone material (0.0193M) had been effectively diluted by 1:100 before being added to the reaction mixture in a 1 in 20 dilution, resulting in a final concentration of about 10μM.

Similarly the 5mM daunomycin starting material was diluted by 1:10 before further dilution (1:20) in the PCR reaction mixture. The final concentration in this case was 25μM.

Example 3

Identification of further quenching DNA binding agents

Step 1

Identification of absorbers

A number of further DNA duplex binding agents which could be used to absorb fluorescent energy were identified using the following methodology.

A tenfold dilution series of the potential quencher was prepared and added in 5μl of each dilution to the PCR reaction mix below.

| | |
|---|---|
| PCR Mix Formulation: | 50μl of 2x Master mix as defined in Example 1 but at 3mM $Mg^{2+}$ |
| | 10μl of Forward Primer (PCO3) at 10μM |
| | 10μl of Reverse Primer (PCO4) at 10μM |
| | 5μl of Sybr Gold |
| | 10μl of HPLC Grade Water |
| | 5μl of Dark Quencher at various concentrations |

This was then subjected to an amplification reaction as described in Example 1. The purpose of this experiment is two fold, firstly it establishes if the inclusion of the potential quencher in the mix will inhibit the PCR and if at what concentrations it does so. Secondly we can see if the inclusion of the potential quencher in the mix reduces (quenches) the fluorescence of the Sybr Gold (By comparison of the baseline and maximum fluorescence for the run with a control that does not contain quencher). Between them these two results allow the determiniation of a concentration range at which the potential molecule could be particularly useful as a DNA duplex binding agent, which can act as a dark quencher.

Reduction of the Sybr Gold™ signal may be due however to the potential quencher out-competing the Sybr Gold for binding sites in the minor groove. Although difficult to tell the difference between this and quenching (or perhaps both together) it is also a beneficial observation, it would mean the potential quencher can indeed intercalate.

Potential quenchers which were identified in this way were then subjected to the following experiments to clarify this.

Step 2

Test the potential molecule in the full Dark Quencher format with a FAM labelled probe

Using the narrower concentration range for the potential quencher established in experiment 1), 5μl of the selected potential quenchers was added to the following mix:

| | |
|---|---|
| PCR Mix Formulation: | 50μl of 2x Master mix at 3mM $Mg^{2+}$ |
| | 10μl of Forward Primer (PC03) at 10μM |
| | 10μl of Reverse Primer (PCO4) at 10μM |
| | 10μl of Probe (CTW19) at 2μM |
| | 5μl of HPLC Grade Water |
| | 5μl of Dark Quencher at various concentrations narrowed down by experiment 1) |

This was then subjected to amplification as described in Example 1, and fluorescence monitored. Those quenchers which produced results of the type illustrated in Figure 6, with a good portion of exponential linearity were selected for further evalutation.

If no effect was observed, the potential quenchers were reserved for further testing using alternative dyes such as Cy3(which fluoresces at 565nm) and Cy5.5 (which fluoresces at 694nm).

The baseline adjustment function of PCR machines will skew the curve (as it is in Figure 6) as they subtract from the 'wrong' end of the reaction. This can be corrected by exporting the raw data and applying a baseline adjustment formula that has been adjusted to deal with decreases rather than rises in fluorescence as outlined above.

Step 3

Quantifying the effect

Potential quenchers which were successful in test 2 were included in a test with a 10-fold dilution series of target DNA. A 3.3 cycle difference in the CT values between subsequent dilutions showed that the effect was directly linked to the amount of target DNA and therefore the PCR process as well.

| | |
|---|---|
| PCR Mix Formulation: | 50μl of 2x Master mix at 3mM $Mg^{2+}$ |
| | 10μl of Forward Primer (PCO3) at 10μM |
| | 10μl of Reverse Primer (PCO4) at 10μM |
| | 10μl of Probe (CTW19) at 2μM |
| | 5μl of HPLC Grade Water |
| | 5μl of Dark Quencher at concentration now defined by experiment 1) and 2) |

This mix was then amplified as described in Example 1, with the variable subject to change being the concentration of the target DNA (our 10-fold series). Only one set of non-target controls (NTCs) was run.
Using this protocol, mitoxantrone, daunomycin, Draq5™ and Apoptrak™ were identified as useful dark quenchers.

**Claims**

**1.** A method for detecting the presence of a target nucleic acid sequence in a sample, said method comprising:

(a) adding to a sample suspected of containing said target nucleic acid sequence, a fluorescently labelled probe specific for said target sequence, and a DNA duplex binding agent which can absorb fluorescent energy from the fluorescent label on the probe but whose emissions are not detectable in the context of the method,
(b)subjecting the thus formed mixture to an amplification reaction in which target nucleic acid is amplified,
(c) subjecting said sample to conditions under which the said probe hybridises to the target sequence, and
(d) monitoring fluorescence from said sample; wherein the probe is released intact from the target sequence.

**2.** A method according to claim 1 wherein the DNA duplex binding agent has a fused conjugated ring system.

**3.** A method according to claim 1 or claim 2 wherein the DNA duplex binding agent is mitoxantrone (1,4-dihydroxy 5,8-bis[[2-[(2-hydroxyethyl)amino]ethyl]amino]-9,10-anthracenedione) or it salt such as the hydrochloride or dihydrochloride salt, nogalamycin (2R- (2α, 3β, 4α, 5β,-6α,11β,13α,14α)]-11-[6-deoxy-3-C-methyl-2,3,4-tri-O-methyl-α-L-mannopyranosyl) oxy]- 4-(dimethylamino)- 3,4,5,6,9,11,12,13,14,16- decahydro- 3,5,8,10,13- pentahydroxy- 6,13-dimethyl-9,16-dioxo-2,6-epoxy-2H-naphthaceno[1,2-b]oxocin-14-carboxylic acid methyl easter) or daunomycin (8S,-cis)-8-acetyl-10-[3-amino-2,3,6-trideoxy-α-L-lyxo-hexopyranosyl)oxy]-7,8,9,10-tetrahydro-6,8,11-trihydroxy-1-methoxy-5,12-naphthacendione).

**4.** A method according to claim 3 wherein the DNA binding agent is mitoxantrone.

**5.** A method according to claim 1 or claim 2 wherein the DNA binding agent is a DNA binding compound of formula (I)

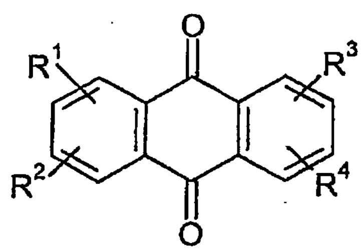

(I)

where $R^1$, $R^2$, $R^3$ and $R^4$ are independently selected from hydrogen, a functional group selected from halo, cyano, nitro, oxo, -OC (O) $R^a$, $-OR^a$, $-C(O)OR^a$, $S(O)_tR^a$, $NR^bR^c$, OC (O) $NR^bR^c$ $C(O)NR^bR^c$=, OC (O) $NR^bR^c$, $-NR^a$-$CONR^bR^c$, $-C=NOR^a$, $-N=CR^bR^c$, S (O) $_tNR^bR^c$, C (S) $R^a$, C (S) $OR^a$, $C(S)NR^bR^c$ or $-NR^bS$ (O) $_tR^a$ where $R^a$, $R^b$ and $R^c$ are independently selected from hydrogen or optionally substituted hydrocarbyl, or $R^b$ and $R^c$ together form an optionally substituted ring which optionally contains further heteroatoms such as S (O) $_s$, oxygen and nitrogen, n is an integer of 1 or 2, s is 0, 1 or 2, t is 0 or an integer of 1-3, or a hydrocarbyl group optionally substituted by functional groups as defined above, or $R^1$ and $R^2$ or $R^3$ and $R^4$ are optionally joined together to form a ring which optionally contains heteroatoms, and/or is optionally substituted by a functional group as defined above, or a hydrocarbyl group.

**6.** A method according to claim 5 wherein the compound of formula (I) is a compound of formula (IA)

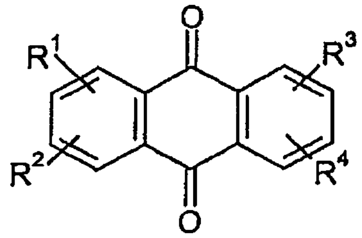

(IA)

wherein $R^1$, $R^2$, $R^3$ and $R^4$ are independently selected from hydrogen, X, NH-ANHR and NH-A-N(O)R'R" where X is hydroxy, halo, amino, $C_{1-4}$alkoxy or $C_{2-8}$alkanoyloxy, A is a $C_{2-4}$alkylene group with a chain length between NH and NHR or N(O)R'R" of at least 2 carbon atoms and R, R' and R" are each independently selected from $C_{1-4}$alkyl and $C_{2-4}$hydroxyalkyl and $C_{2-4}$dihydroxyalkyl, provided that a carbon atom attached to a nitrogen atom does not carry a hydroxy group and that no carbon atom is substituted by two hydroxy groups; or R' and R" together are a $C_{2-6}$alkylene group which, with the nitrogen atom to which R' and R" are attached form a heterocyclic ring having 3 to 7 atoms, with the proviso that at least one of $R^1$, $R^2$, $R^3$ and $R^4$ is a group NHR-A-N(O)R'R".

**7.** A method according to claim 1 wherein the DNA duplex binding agent does not emit visible light.

**8.** A method according to any one of the preceding claims wherein the target nucleic acid is rendered single stranded prior to hybridisation to the probe in step (c).

**9.** A method according to any one of the preceding claims wherein the amplification reaction is the polymerase chain reaction (PCR)

**10.** A method according to any one of the preceding claims wherein the probe hybridises with the target nucleic acid during every cycle of the amplification reaction.

**11.** A method according to claim 10 wherein the fluorescence from the sample is monitored throughout the amplification reaction.

**12.** A method according to claim 11 wherein fluorescence data generated is used to determine the rates of probe hybridisation.

**13.** A method according to any one of claims 10 to 12 wherein the fluorescence data is used to quantitate the amount of target nucleic acid present in the sample.

**14.** A method according to any one of the preceding claims wherein the fluorescent label is a rhodamine dye, Cy5, fluorescein or a fluorescein derivative.

**15.** A method according to any one of the preceding claims wherein the fluorescent label is attached at an end region of the probe.

**16.** A method according to claim 15 wherein the fluorescent label is attached at the 3'end of the probe and prevents

extension thereof by a polymerase.

**17.** A method according to anyone of the preceding claims wherein the probe is designed such that it is released intact from the target sequence during a phase of the amplification process other than the extension phase.

**18.** A method according to any one of claims 1 to 16 wherein the probe is released intact from the target sequence during the extension phase of the amplification process by the action of the polymerase, and the amplification reaction is effected using a polymerase which lacks 5'-3' exonuclease activity.

**19.** A method according to claim 1 which comprises performing nucleic acid amplification on a target polynucleotide in the presence of (a) a nucleic acid polymerase (b)at least one primer capable of hybridising to said target polynucleotide, (c) an oligonucleotide probe which is capable of binding to said target polynucleotide sequence and which contains a fluorescent label and (d) a DNA duplex binding agent which is capable of absorbing fluorescent energy from the said fluorescent label, and whose emissions are not detectable in the context of the method; and monitoring changes in fluorescence during the amplification reaction.

**20.** A method according to claim 19 wherein the amplification is suitably carried out using a pair of amplification primers.

**21.** A method according to claim 19 or claim 20 wherein the nucleic acid polymerase is a thermostable polymerase.

**22.** A method according to any one of the preceding claims wherein in a further step, a hybridisation assay is carried out and a hybridisation condition which is characteristic of the sequence is measured.

**23.** A method according to claim 22 wherein the condition is temperature, electrochemical potential, or reaction with an enzyme or chemical.

**24.** A method according to claim 23 wherein the condition is temperature.

**25.** A method according to claim 24 which is used to detect allelic variation or a polymorphism in a target sequence.

**26.** A method for determining a characteristic of a sequence, said method comprising;

a) adding to a sample suspected of containing said sequence, a fluorescently labelled probe specific for said target sequence and a DNA duplex binding agent able to absorb fluorescence from a fluorescent label on the probe and whose emissions are not detectable in the context of the method,
(b) subjecting said sample to conditions under which the said probe hybridises to the target sequence and thereafter is released intact from the target sequence,
(c) monitoring fluorescence from said sample and determining a particular reaction condition, characteristic of said sequence, at which fluorescence changes as a result of the release intact of the probe from the target nucleic acid sequence.

**27.** A method according to claim 26 wherein the reaction condition characteristic of said sequence is temperature, electrochemical potential, or reaction with an enzyme or chemical.

**28.** A method according to claim 27 wherein the condition is temperature.

**29.** A method according to any one of claims 26 to 28 wherein the results obtained from two sequences are compared in order to determine the presence of polymorphisms or variations therebetween.

**30.** A method according to any one of claims 26 to 29 wherein the DNA duplex binding agent is mitoxantrone (1,4-dihydroxy 5,8-bis[[2-[(2-hydroxyethyl)amino]ethyl] amino]-9,10-anthracenedione) or it salt such as the hydrochloride or dihydrochloride salt, nogalamycin (2R-(2α,3β, 4α, 5β, 6α, 11β, 13a, 14α)] -11-[6-deoxy-3-C-mehtyl-2,3,4-tri-O-methyl-α-L-mannopyranosyl) oxy]-4-(dimethylamino)-3,4,5,6,9,11,12,13,14,16-decahydro-3,5,8,10,13-pentahydroxy-6,13-dimethyl-9,16-dioxo-2,6-epoxy-2H-naphthaceno[1,2-b]oxocin-14-carboxylic acid methyl ester) or daunomycin (85,-cis)-8-acetyl-10-[3-amino-2,3,6-trideoxy-α-L-lyxo-hexopyranosyl)oxyl-7,8,9,10-tetrahydro-6,8,11-trihydroxy-1-methoxy-5,12-naphthacendione).

**31.** A method according to any one of claim 26 to 29, wherein the DNA duplex binding agent is a compound of formula

(IA) as defined in claim 6.

**Patentansprüche**

1. Verfahren zum Nachweisen der Anwesenheit einer Zielnucleinsäuresequenz in einer Probe, wobei das Verfahren folgendes umfasst:

(a) man versetzt eine Probe, von der vermutet wird, dass sie die Zielnucleinsäuresequenz enthält, mit einer fluoreszierend markierten, für die Zielsequenz spezifischen Sonde und einem DNA-Duplex-Bindungsmittel, das Fluoreszenzenergie von der fluoreszierenden Markierung an der Sonde absorbieren kann, dessen Emissionen aber in Verbindung mit dem Verfahren nicht nachweisbar sind,
(b) man unterwirft das auf diese Weise gebildete Gemisch einer Amplifikationsreaktion, bei der die Zielnucleinsäure amplifiziert wird,
(c) man unterwirft die Probe Bedingungen, unter denen die Sonde mit der Zielsequenz hybridisiert, und
(d) man überwacht die Fluoreszenz der Probe, wobei die Sonde aus der Zielsequenz in intakter Form freigesetzt wird.

2. Verfahren nach Anspruch 1, wobei das DNA-Duplex-Bindungsmittel ein fusioniertes, konjugiertes Ringsystem aufweist.

3. Verfahren nach Anspruch 1 oder 2, wobei es sich beim DNA-Duplex-Bindungsmittel um Mitoxantron (1,4-Dihydroxy-5,8-bis-[[2-[(2-hydroxyethyl)-amino]-ethyl]-amino]-9,10-anthracendion) oder ein Salz davon, wie das Hydrochlorid- oder Dihydrochloridsalz, Nogalamycin (2R-(2α,3β,4,5β,6α,11β,13α,14α)]-11-[6-desoxy-3-C-methyl-2,3,4-tri-O-methyl-α-L-mannopyranosyl)-oxy]-4-(dimethylamino)-3,4,5,6,9,11,12,13,14,16-decahydro-3,5,8,10,13-pentahydroxy-6,13-dimethyl-9,16-dioxo-2,6-epoxy-2H-naphthaceno[1,2-b]oxocin-14-carbonsäure-methylester) oder Daunomycin (8S,cis)-8-Acetyl-10-[3-amino-2,3,6-tridesoxy-α-L-lyxo-hexopyranosyl)-oxy]-7,8,9,10-tetrahydro-6,8,11 -trihydroxy-1-methoxy-5,12-naphthacendion) handelt.

4. Verfahren nach Anspruch 3, wobei es sich beim DNA-Bindungsmittel um Mitoxantron handelt.

5. Verfahren nach Anspruch 1 oder 2, wobei es sich beim DNA-Bindungsmittel um eine DNA bindende Verbindung der Formel (I) handelt

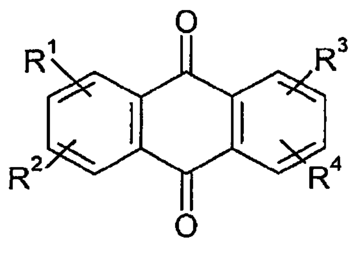

(I)

wobei $R^1$, $R^2$, $R^3$ und $R^4$ unabhängig voneinander aus Wasserstoff und einer funktionellen Gruppe ausgewählt sind, die aus Halogen, Cyano, Nitro, Oxo, $-OC(O)R^a$, $-OR^a$, $-C(O)OR^a$, $S(O)_tR^a$, $NR^bR^c$, $OC(O)NR^bR^c$, $C(O)NR^bR^c$, $OC(O)NR^bR^c$, $-NR^aCONR^bR^c$, $-C{=}NOR^a$, $-N{=}CR^bR^c$, $S(O)_tNR^bR^c$, $C(S)_nR^a$, $C(S)OR^a$, $C(S)NR^bR^c$ oder $-NR^bS(O)_tR^a$ ausgewählt ist, wobei $R^a$, $R^b$ und $R^c$ unabhängig voneinander ausgewählt sind aus Wasserstoff oder gegebenenfalls substituiertem Hydrocarbyl oder $R^b$ und $R^c$ zusammen einen gegebenenfalls substituierten Ring bilden, der gegebenenfalls weitere Heteroatome, wie $S(O)_s$, Sauerstoff und Stickstoff enthält, n eine ganze Zahl mit einem Wert von 1 oder 2 bedeutet, s den Wert 0, 1 oder 2 hat, t den Wert 0 hat oder eine ganze Zahl mit einem Wert von 1-3 bedeutet, oder eine Hydrocarbylgruppe bilden, die gegebenenfalls durch funktionelle Gruppen gemäß der vorstehenden Definition substituiert sind, oder $R^1$ und $R^2$ oder $R^3$ und $R^4$ gegebenenfalls miteinander einen Ring bilden, der gegebenenfalls Heteroatome enthält und/oder gegebenenfalls durch eine funktionelle Gruppe gemäß der vorstehenden Definition oder eine Hydrocarbylgruppe substituiert ist.

6. Verfahren nach Anspruch 5, wobei es sich bei der Verbindung der Formel (I) um eine Verbindung der Formel (IA)

handelt

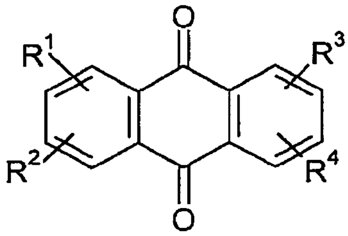

(IA)

wobei $R^1$, $R^2$, $R^3$ und $R^4$ unabhängig voneinander aus Wasserstoff, X, NH-ANHR und NH-A-N(O)R'R" ausgewählt sind, wobei X Hydroxy, Halogen, Amino, $C_{1-4}$-Alkoxy oder $C_{2-8}$-Alkanoyloxy bedeutet, A eine $C_{2-4}$-Alkylengruppe mit einer Kettenlänge zwischen NH und NHR oder N(O)R'R" von mindestens 2 Kohlenstoffatomen bedeutet und R, R' und R" jeweils unabhängig voneinander aus $C_{1-4}$-Alkyl und $C_{2-4}$-Hydroxyalkyl und $C_{2-4}$-Dihydroxyalkyl ausgewählt sind, mit der Maßgabe, dass ein Kohlenstoffatom, das an ein Stickstoffatom gebunden ist, keine Hydroxygruppe trägt und dass kein Kohlenstoffatom durch 2 Hydroxygruppen substituiert ist; oder R' und R" zusammen eine $C_{2-6}$-Alkylengruppe darstellen, die mit dem Stickstoffatom, an das R' und R" gebunden sind, einen heterocyclischen Ring mit 3 bis 7 Atomen bilden, mit der Maßgabe, dass es sich bei mindestens einem der Reste $R^1$, $R^2$, $R^3$ und $R^4$ um eine NH-AN(O)R'R"-Gruppe handelt.

**7.** Verfahren nach Anspruch 1, wobei das DNA-Duplex-Bindungsmittel kein sichtbares Licht emittiert.

**8.** Verfahren nach einem der vorstehenden Ansprüche, wobei die Zielnucleinsäure vor der Hybridisierung mit der Sonde in Stufe (c) einzelsträngig gemacht wird.

**9.** Verfahren nach einem der vorstehenden Ansprüche, wobei es sich bei der Amplifikationsreaktion um die Polymerase-Kettenreaktion (PCR) handelt.

**10.** Verfahren nach einem der vorstehenden Ansprüche, wobei die Sonde während eines jeden Zyklus der Amplifikationsreaktion mit der Zielnucleinsäure hybridisiert.

**11.** Verfahren nach Anspruch 10, wobei die von der Probe ausgehende Fluoreszenz während der gesamten Amplifikationsreaktion überwacht wird.

**12.** Verfahren nach Anspruch 11, wobei die erzeugten Fluoreszenzdaten zur Bestimmung der Geschwindigkeiten der Sondenhybridisierung verwendet werden.

**13.** Verfahren nach einem der Ansprüche 10 bis 12, wobei die Fluoreszenzdaten zur quantitativen Bestimmung der Menge der in der Probe vorhandenen Zielnucleinsäure verwendet werden.

**14.** Verfahren nach einem der vorstehenden Ansprüche, wobei es sich bei der fluoreszierenden Markierung um einen Rhodaminfarbstoff, Cy5, Fluorescein oder ein Fluoresceinderivat handelt.

**15.** Verfahren nach einem der vorstehenden Ansprüche, wobei die fluoreszierende Markierung an einem Endbereich der Sonde gebunden wird.

**16.** Verfahren nach Anspruch 15, wobei die fluoreszierende Markierung am 3'-Ende der Sonde gebunden wird und deren Erweiterung durch eine Polymerase verhindert.

**17.** Verfahren nach einem vorstehenden Ansprüche, wobei die Sonde so konzipiert ist, dass sie während einer Phase des Amplifikationsvorgangs, bei dem es sich nicht um die Erweiterungsphase handelt, in intakter Form aus der Zielsequenz freigesetzt wird.

**18.** Verfahren nach einem der Ansprüche 1 bis 16, wobei die Sonde während der Erweiterungsphase des Amplifikationsvorgangs durch die Einwirkung der Polymerase in intakter Form aus der Zielsequenz freigesetzt wird und die

Amplifikationsreaktion unter Verwendung einer Polymerase, die keine 5'-3'-Exonuclease-Aktivität aufweist, durchgeführt wird.

**19.** Verfahren nach Anspruch 1, umfassend die Durchführung einer Nucleinsäure-Amplifikation an einem Zielpolynucleotid in Gegenwart von (a) einer Nucleinsäure-Polymerase, (b) mindestens eines Primers, der zur Hybridisierung mit dem Zielpolynucleotid befähigt ist, (c) einer Oligonucleotidsonde, die zur Bindung an die Zielpolynucleotidsequenz befähigt ist und die eine fluoreszierende Markierung enthält, und (d) eines DNA-Duplex-Bindungsmittels, das zur Absorption von Fluoreszenzenergie der fluoreszierenden Markierung befähigt ist und dessen Emissionen in Verbindung mit dem Verfahren nicht nachweisbar sind; und das Überwachen von Änderungen der Fluoreszenz während der Amplifikationsreaktion.

**20.** Verfahren nach Anspruch 19, wobei die Amplifikation in geeigneter Weise unter Verwendung eines Paars von Amplifikationsprimern durchgeführt wird.

**21.** Verfahren nach Anspruch 19 oder 20, wobei es sich bei der Nucleinsäure-Polymerase um eine thermostabile Polymerase handelt.

**22.** Verfahren nach einem der vorstehenden Ansprüche, wobei in einer weiteren Stufe ein Hybridisierungstest durchgeführt wird und ein Hybridisierungszustand, der für die Sequenz charakteristisch ist, gemessen wird.

**23.** Verfahren nach Anspruch 22, wobei es sich bei dem Zustand um die Temperatur, das elektrochemische Potential oder die Umsetzung mit einem Enzym oder einer Chemikalie handelt.

**24.** Verfahren nach Anspruch 23, wobei es sich bei dem Zustand um die Temperatur handelt.

**25.** Verfahren nach Anspruch 24, das zum Nachweis einer Allelenvariation oder eines Polymorphismus in einer Zielsequenz verwendet wird.

**26.** Verfahren zur Bestimmung einer charakteristischen Eigenschaft einer Sequenz, wobei das Verfahren folgendes umfasst:

a) man versetzt eine Probe, von der vermutet wird, dass sie die Sequenz enthält, mit einer fluoreszierend markierten, für die Zielsequenz spezifischen Sonde und einem DNA-Duplex-Bindungsmittel, das Fluoreszenz von einer fluoreszierenden Markierung an der Sonde absorbieren kann und dessen Emissionen in Verbindung mit dem Verfahren nicht nachweisbar sind,
(b) man unterwirft die Probe Bedingungen, unter denen die Sonde mit der Zielsequenz hybridisiert und anschließend in intakter Form von der Zielsequenz freigesetzt wird, und
(c) man überwacht die Fluoreszenz der Probe und bestimmt einen speziellen Reaktionszustand, der für die Sequenz charakteristisch ist, bei dem sich die Fluoreszenz als Folge der Freisetzung der Sonde in intakter Form aus der Zielnucleinsäuresequenz verändert.

**27.** Verfahren nach Anspruch 26, wobei es sich bei dem für die Sequenz charakteristischen Reaktionszustand um die Temperatur, das elektrochemische Potential oder die Umsetzung mit einem Enzym oder einer Chemikalie handelt.

**28.** Verfahren nach Anspruch 27, wobei es sich bei dem Zustand um die Temperatur handelt.

**29.** Verfahren nach einem der Ansprüche 26 bis 28, wobei die von 2 Sequenzen erhaltenen Ergebnisse verglichen werden, um zwischen ihnen vorliegende Polymorphismen oder Variationen festzustellen.

**30.** Verfahren nach einem der Ansprüche 26 bis 29, wobei es sich beim DNA-Duplex-Bindungsmittel um Mitoxantron (1,4-Dihydroxy-5,8-bis-[[2-[(2-hydroxyethyl)-amino]-ethyl]-amino]-9,10-anthracendion) oder ein Salz davon, wie das Hydrochlorid- oder Dihydrochloridsalz, Nogalamycin (2R-(2α,3β,4α,5β,6α,11β,13α,14α)]-11-[6-desoxy-3-C-methyl- 2,3,4- tri- O- methyl- α- L- mannopyranosyl)-oxy]- 4-(dimethylamino)- 3,4,5,6,9,11,12,13,14,16- decahydro-3,5,8,10,13-pentahydroxy-6,13-dimethyl-9,16-dioxo-2,6-epoxy-2H-naphthaceno[1,2-b]oxocin-14-carbonsäuremethylester) oder Daunomycin (8S,cis)-8-Acetyl-10-[3-amino-2,3,6-tridesoxy-α-L-lyxo-hexopyranosyl)-oxy]-7,8,9,10-tetrahydro-6,8,11-trihydroxy-1-methoxy-5,12-naphthacendion) handelt.

**31.** Verfahren nach einem der Ansprüche 26 bis 29, wobei es sich beim DNA-Duplex-Bindungsmittel um eine Verbindung

der Formel (IA) gemäß der Definition in Anspruch 6 handelt.

## Revendications

1. Procédé permettant de détecter la présence d'une séquence d'acides nucléiques cible dans un échantillon, ledit procédé comprenant :

   (a) l'ajout à un échantillon suspecté de contenir ladite séquence d'acides nucléiques cible d'une sonde contenant un marqueur fluorescent spécifique de ladite séquence cible et d'un agent se liant à un ADN double brin pouvant absorber l'énergie de fluorescence provenant du marqueur fluorescent se trouvant sur la sonde, mais dont les émissions ne sont pas détectables dans le contexte du procédé,
   (b) le fait de soumettre le mélange ainsi formé à une réaction d'amplification dans laquelle l'acide nucléique cible est amplifié,
   (c) le fait de soumettre ledit échantillon à des conditions dans lesquelles ladite sonde s'hybride à la séquence cible, et
   (d) la surveillance de la fluorescence provenant dudit échantillon ; dans lequel la sonde reste intacte après sa libération de la séquence cible.

2. Procédé selon la revendication 1, dans lequel l'agent se liant à un ADN double brin contient un système de cycles fusionnés et conjugués.

3. Procédé selon la revendication 1 ou la revendication 2, dans lequel l'agent se liant à un ADN double brin est la mitoxantrone (1,4-dihydroxy-5,8-bis[[2-[(2-hydroxyéthyl)-amino]éthyl]amino]-9,10-anthracènedione) ou son sel, comme le sel de chlorhydrate ou de dichlorhydrate, la nogalamycine (2R- (2α, 3β, 4α, 5β, 6α, 11β, 13α, 14α) ] -11-[ 6-désoxy-3-C-méthyl-2,3,4-tri-O-méthyl-α-L-mannopyranosyl)-oxy]-4-(diméthylamino) -3, 4, 5, 6, 9, 11, 12, 13, 14, 16-décahydro-3,5,8,10,13-pentahydroxy-6,13-diméthyl-9,16-dioxo-2,6-époxy-2H-naphthacéno[1,2-b]oxocin-14-carboxylate de méthyle) ou la daunomycine (8S,-cis)-8-acétyl-10-[3-amino-2,3,6-tridésoxy-α-L-lyxo-hexopyranosyl) oxy]-7,8,9,10-tétrahydro-6,8,11-trihydroxy-1-méthoxy-5,12-naphthacènedione.

4. Procédé selon la revendication 3, dans lequel l'agent se liant à un ADN est la mitoxantrone.

5. Procédé selon la revendication 1 ou la revendication 2, dans lequel l'agent se liant à un ADN est un composé se liant à un ADN de formule (I)

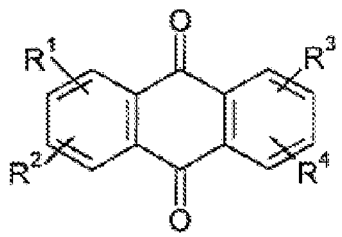

(I)

dans laquelle $R^1$, $R^2$, $R^3$ et $R^4$ sont indépendamment choisis parmi un hydrogène, un groupe fonctionnel choisi parmi un halogène, un cyano, un nitro, un oxo, -OC (O) $R^a$, -$OR^a$, -C (O) $OR^a$, $S(O)_tR^a$, $NR^bR^c$, OC (O) $NR^bR^c$, C (O) $NR^bR^c$, OC (O) $NR^bR^c$, -$NR^aCONR^bR^c$, -C=$NOR^a$, -N=$CR^bR^c$, S (O) $_tNR^bR^c$, C (S) $_nR^a$, C (S) $OR^a$, C(S)$NR^bR^c$ ou -$NR^bS$ (O) $_tR^a$ où $R^a$, $R^b$ et $R^c$ sont indépendamment choisis parmi un hydrogène ou un hydrocarbyle facultativement substitué, ou $R^b$ et $R^c$ forment ensemble un cycle facultativement substitué qui contient facultativement des hétéroatomes supplémentaires, tels que S(O)s, un oxygène et un azote, n est un nombre entier égal à 1 ou 2, S est égal à 0, 1 ou 2, t est égal à 0 ou est un nombre allant de 1 à 3, ou un groupe hydrocarbyle facultativement substitué avec des groupes fonctionnels tels que définis ci-dessus, ou $R^1$ et $R^2$ ou $R^3$ et $R^4$ sont facultativement liés l'un à l'autre pour former un cycle qui contient facultativement des hétéroatomes et/ou qui est facultativement substitué avec un groupe fonctionnel tel que défini ci-dessus ou un groupe hydrocarbyle.

**6.** Procédé selon la revendication 5, dans lequel le composé de formule (I) est un composé de formule (IA)

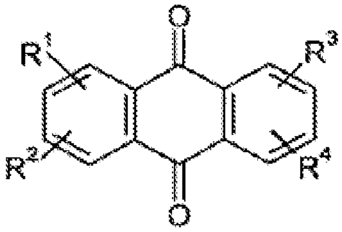

(IA)

dans laquelle $R^1$, $R^2$, $R^3$ et $R^4$ sont indépendamment choisis parmi un hydrogène, X, NH-ANHR et NH-A-N(O)R'R " où X est un hydroxy, un halogène, un amino, un alcoxy en $C_1$ à $C_4$ ou un alcanoyloxy en $C_2$ à $C_8$, A est un groupe alkylène en $C_2$ à $C_4$ ayant une longueur de chaîne entre NH et NHR ou N(O)R'R" d'au moins 2 atomes de carbone et R, R' et R'' sont chacun indépendamment choisis parmi un alkyle en $C_1$ à $C_4$ et un hydroxyalkyle en $C_2$ à $C_4$ et un dihydroxyalkyle en $C_2$ à $C_4$, à condition qu'un atome de carbone fixé à un atome d'azote ne porte pas un groupe hydroxy et qu'aucun atome de carbone ne soit substitué avec deux groupes hydroxy ; ou R' et R'' sont ensemble un groupe alkylène en $C_2$ à $C_6$ qui, avec l'atome d'azote auquel R' et R'' sont liés former un cycle hétérocyclique contenant 3 à 7 atomes, à condition qu'au moins un parmi $R^1$, $R^2$, $R^3$ et $R^4$ soit un groupe NH-A-N (O) R'R''.

**7.** Procédé selon la revendication 1, dans lequel l'agent se liant à un ADN double brin n'émet pas de lumière visible.

**8.** Procédé selon l'une quelconque des revendications précédentes, dans lequel l'acide nucléique cible est rendu monocaténaire avant l'hybridation de la sonde dans l'étape (c).

**9.** Procédé selon l'une quelconque des revendications précédentes, dans lequel la réaction d'amplification est la réaction en chaîne par polymérase (PCR).

**10.** Procédé selon l'une quelconque des revendications précédentes, dans lequel la sonde s'hybride à l'acide nucléique cible lors de chaque cycle de la réaction d'amplification.

**11.** Procédé selon la revendication 10, dans lequel la fluorescence provenant de l'échantillon est surveillée tout au long de la réaction d'amplification.

**12.** Procédé selon la revendication 11, dans lequel les données de fluorescence générées sont utilisées pour déterminer les taux d'hybridation de la sonde.

**13.** Procédé selon l'une quelconque des revendications 10 à 12, dans lequel les données de fluorescence sont utilisées pour quantifier la quantité d'acide nucléique cible présente dans l'échantillon.

**14.** Procédé selon l'une quelconque des revendications précédentes, dans lequel le marqueur fluorescent est le colorant rhodamine, le Cy5, la fluorescéine ou un dérivé de fluorescéine.

**15.** Procédé selon l'une quelconque des revendications précédentes, dans lequel le marqueur fluorescent est fixé au niveau d'une région terminale de la sonde.

**16.** Procédé selon la revendication 15, dans lequel le marqueur fluorescent est fixé au niveau de l'extrémité 3' de la sonde et empêche son extension par une polymérase.

**17.** Procédé selon l'une quelconque des revendications précédentes, dans lequel la sonde est conçue de manière à être libérée sous forme intacte de la séquence cible lors d'une phase du procédé d'amplification autre que la phase d'extension.

**18.** Procédé selon l'une quelconque des revendications 1 à 16, dans lequel la sonde est libérée sous forme intacte de la séquence cible lors de la phase d'extension du procédé d'amplification sous l'action de la polymérase, et la

réaction d'amplification est effectuée au moyen d'une polymérase n'ayant pas d'activité exonucléase 5'-3'.

**19.** Procédé selon la revendication 1, comprenant la réalisation d'une amplification d'acide nucléique sur un polynucléotide cible en présence (a) d'une polymérase d'acide nucléique, (b) d'au moins une amorce capable de s'hybrider sur ledit polynucléotide cible, (c) d'une sonde oligonucléotidique capable de se lier à ladite séquence polynucléotidique cible et qui contient un marqueur fluorescent et (d) d'un agent se liant à un ADN double brin capable d'absorber l'énergie de fluorescence provenant dudit marqueur fluorescent et dont les émissions ne sont pas détectables dans le contexte du procédé ; et la surveillance des changements de fluorescence lors de la réaction d'amplification.

**20.** Procédé selon la revendication 19, dans lequel l'amplification est réalisée de manière appropriée en utilisant une paire d'amorces d'amplification.

**21.** Procédé selon la revendication 19 ou la revendication 20, dans lequel la polymérase d'acide nucléique est une polymérase thermostable.

**22.** Procédé selon l'une quelconque des revendications précédentes, dans lequel, dans une étape supplémentaire, un essai d'hybridation est réalisé et une condition d'hybridation qui est caractéristique de la séquence est mesurée.

**23.** Procédé selon la revendication 22, dans lequel la condition est la température, un potentiel électrochimique ou une réaction avec une enzyme ou un produit chimique.

**24.** Procédé selon la revendication 23, dans lequel la condition est la température.

**25.** Procédé selon la revendication 24, qui est utilisé pour détecter des variations alléliques ou un polymorphisme dans une séquence cible.

**26.** Procédé permettant de déterminer une caractéristique d'une séquence, ledit procédé comprenant :

(a) l'ajout à un échantillon suspecté de contenir ladite séquence d'une sonde contenant un marqueur fluorescent spécifique de ladite séquence cible et d'un agent se liant à un ADN double brin capable d'absorber la fluorescence provenant d'un marqueur fluorescent se trouvant sur la sonde et dont les émissions ne sont pas détectables dans le contexte du procédé,
(b) le fait de soumettre ledit échantillon à des conditions dans lesquelles ladite sonde s'hybride à la séquence cible puis est libérée sous forme intacte de la séquence cible,
(c) la surveillance de la fluorescence dudit échantillon et la détermination d'une condition réactionnelle particulière, caractéristique de ladite séquence, à laquelle la fluorescence change du fait de la libération de la sonde sous forme intacte de la séquence d'acides nucléiques cible.

**27.** Procédé selon la revendication 26, dans lequel la condition réactionnelle caractéristique de ladite séquence est la température, un potentiel électrochimique ou une réaction avec une enzyme ou un produit chimique.

**28.** Procédé selon la revendication 27, dans lequel la condition est à la température.

**29.** Procédé selon l'une quelconque des revendications 26 à 28, dans lequel les résultats obtenus à partir de deux séquences sont comparés afin de déterminer la présence de polymorphismes ou de variations entre elles.

**30.** Procédé selon l'une quelconque des revendications 26 à 29, dans lequel l'agent se liant à un ADN double brin est la mitoxantrone (1,4-dihydroxy-5,8-bis[[2-[(2-hydroxyéthyl)-amino]éthyl]amino]-9,10-anthracènedione) ou son sel, comme le sel de chlorhydrate ou de dichlorhydrate, la nogalamycine (2R-(2α, 3β, 4α, 5β, 6α, 11β, 13α,14α) ]-11-[6-désoxy-3-C-méthyl-2,3,4-tri-O-méthyl-α-L-mannopyranosyl)-oxy]-4-(diméthylamino) -3, 4, 5, 6, 9, 11, 12, 13, 14,16-décahydro-3,5,8,10,13-pentahydroxy-6,13-diméthyl-9,16-dioxo-2,6-époxy-2H-naphthacéno[1,2-b]oxocin-14-carboxylate de méthyle) ou la daunomycine (8S,-cis)-8-acétyl-10-[3-amino-2,3,6-tridésoxy-α-L-lyxo-hexopyranosyl) oxy]-7,8,9,10-tétrahydro-6,8,11-trihydroxy-1-méthoxy-5,12-naphthacènedione.

**31.** Procédé selon l'une quelconque des revendications 26 à 29, dans lequel l'agent se liant à un ADN double brin est un composé de formule (IA) tel que défini dans la revendication 6.

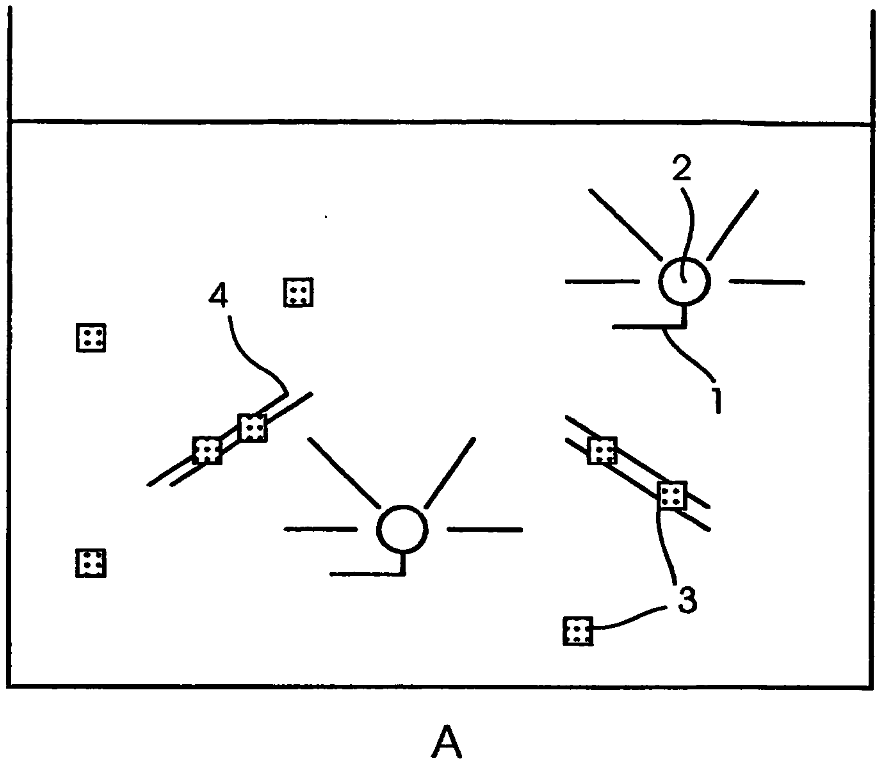


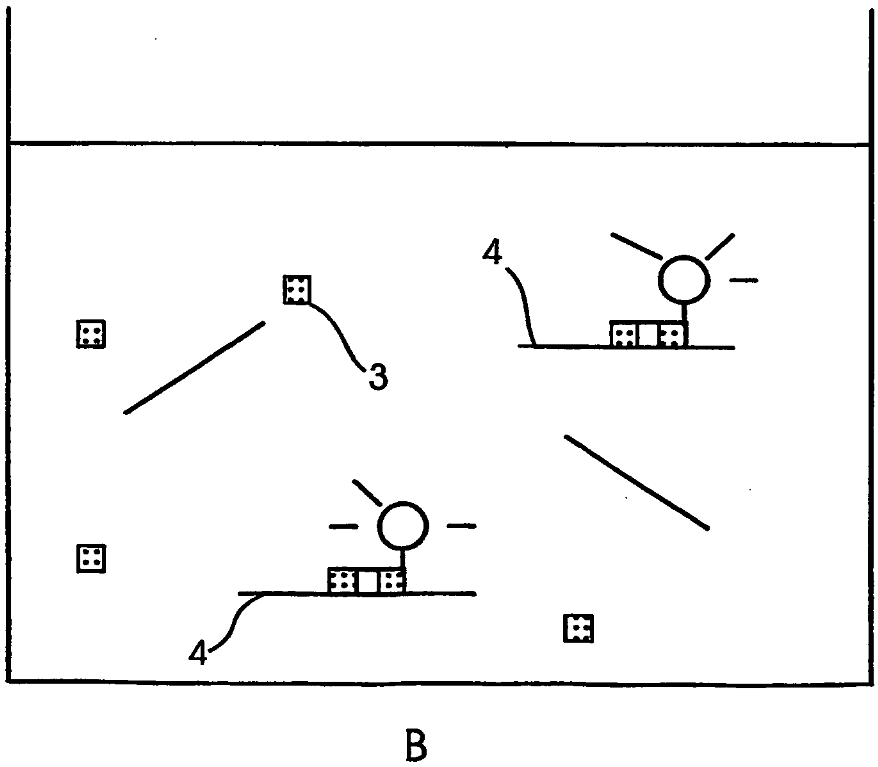


Fig. 1

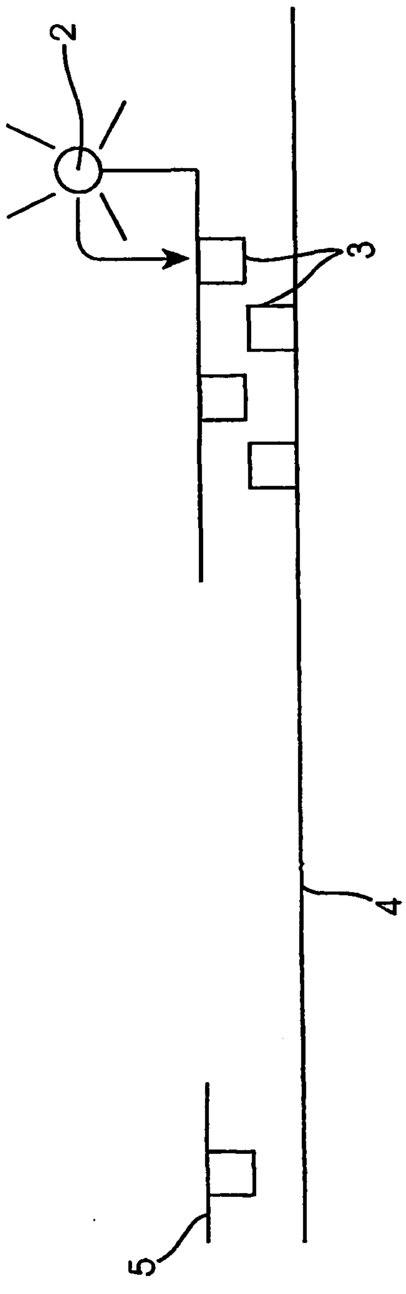


Fig. 2A

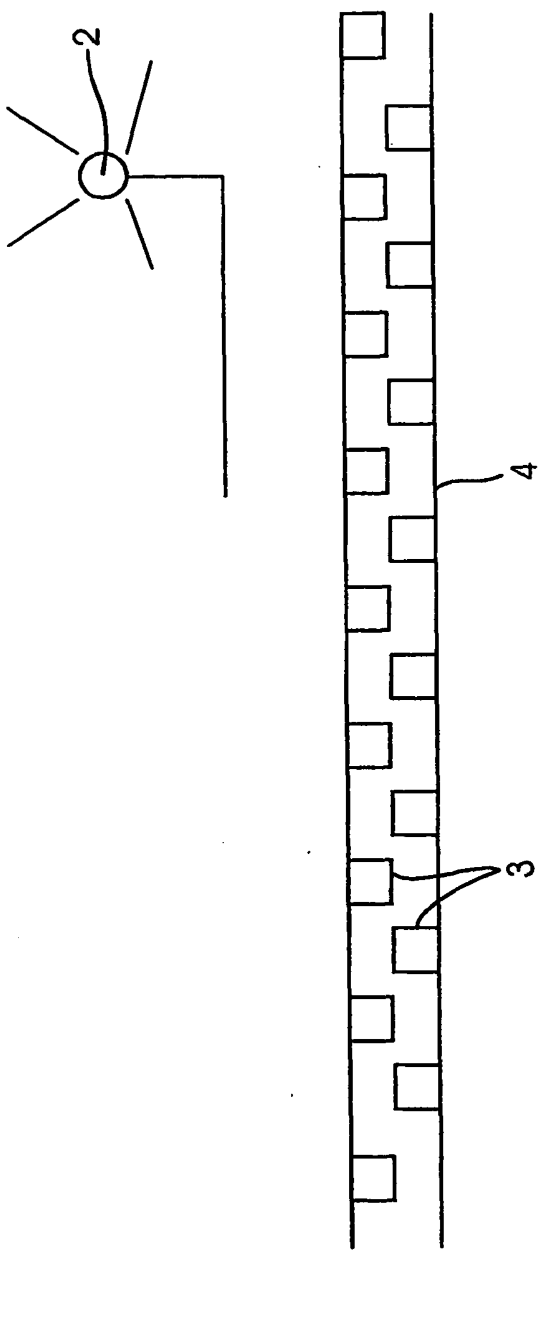


Fig. 2B

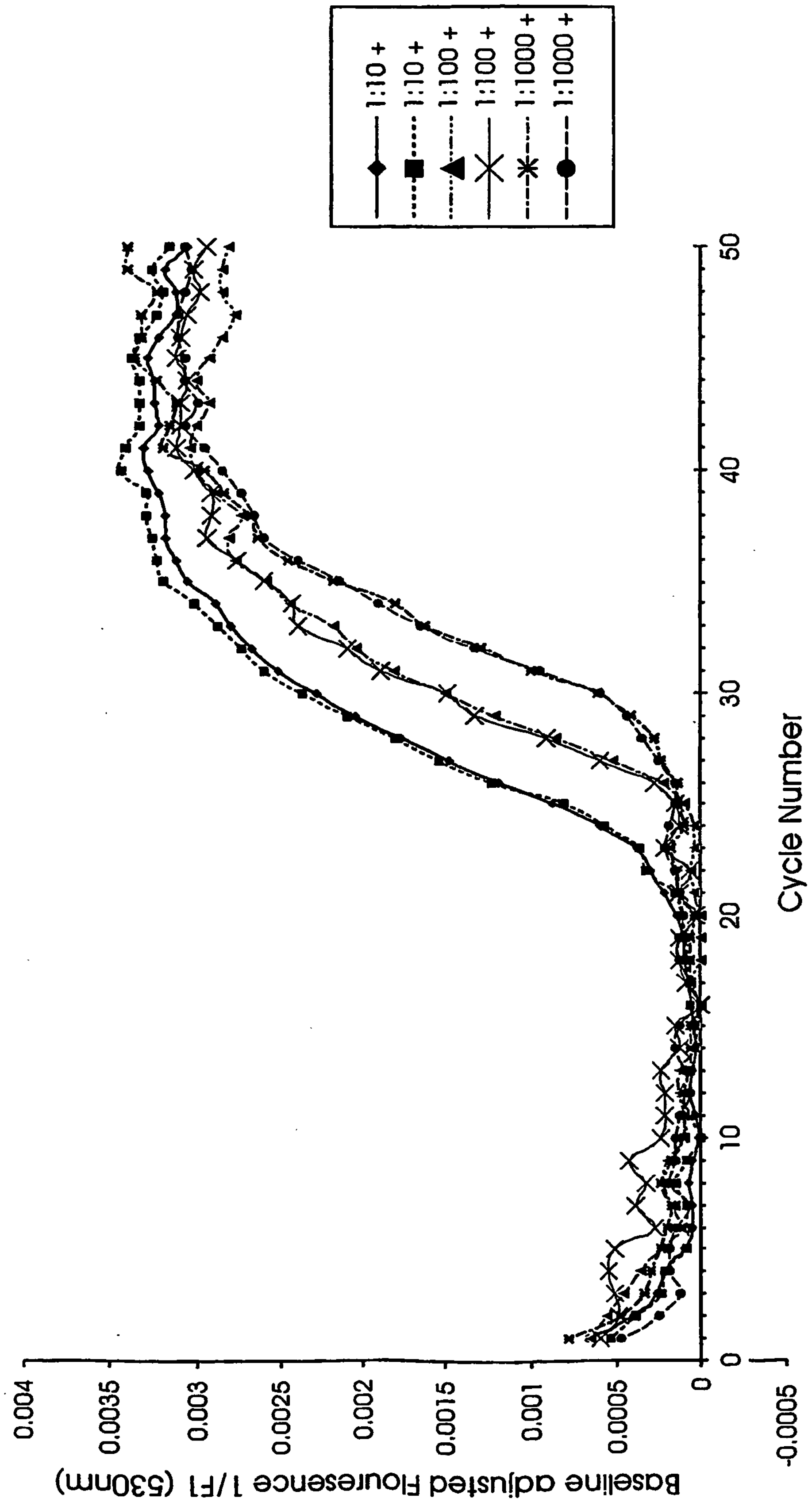

1:10 +
1:10 +
1:100 +
1:100 +
1:1000 +
1:1000 +
Baseline adjusted Flouresence 1/F1 (530nm)
0.004
0.0035
0.003
0.0025
0.002
0.0015
0.001
0.0005
0
-0.0005
0
10
20
30
40
50
Cycle Number


Fig. 3

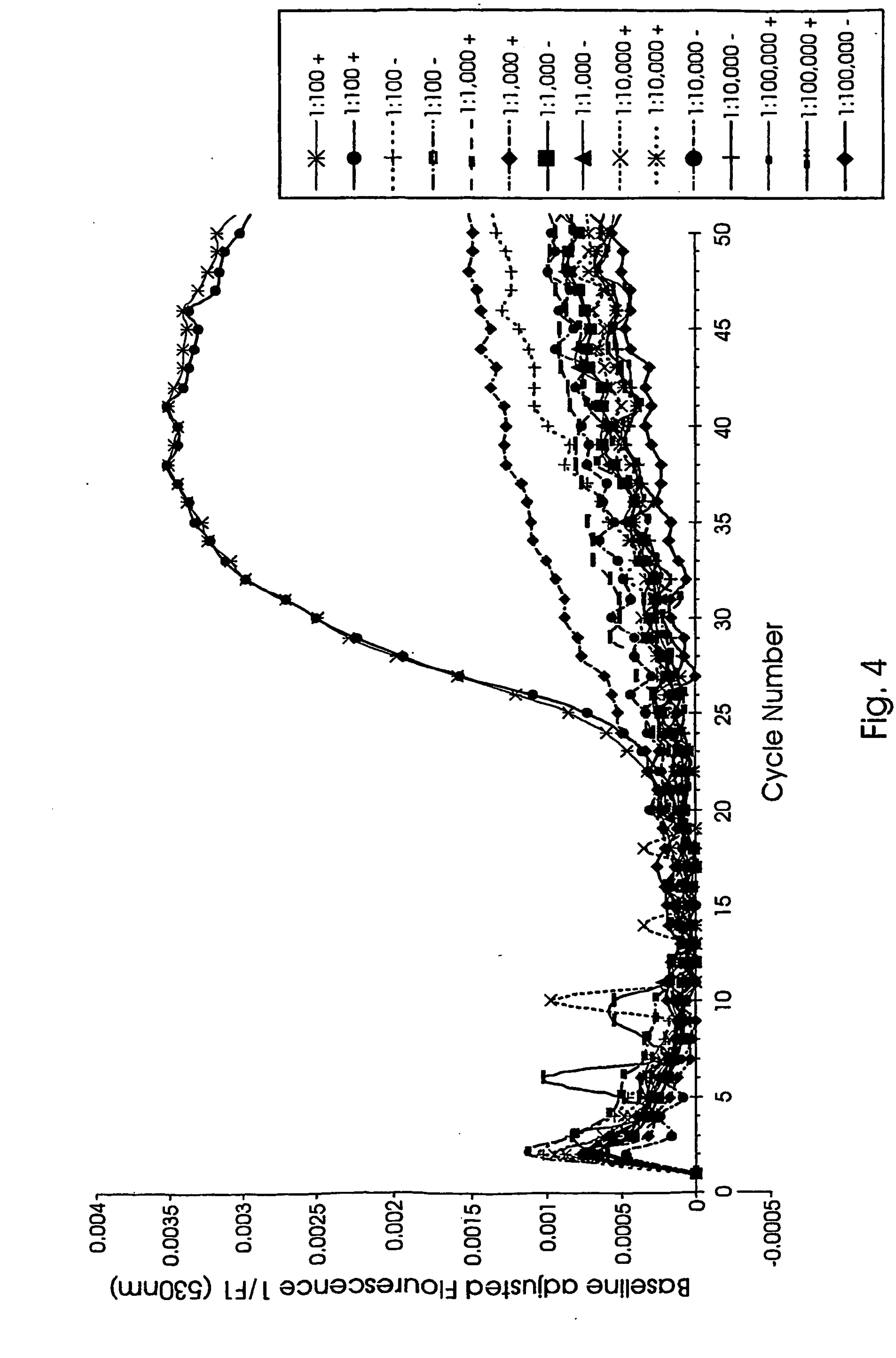
Baseline adjusted Flouresecence 1/F1 (530nm)
0.004
0.0035
0.003
0.0025
0.002
0.0015
0.001
0.0005
0
-0.0005
Cycle Number
0
5
10
15
20
25
30
35
40
45
50
1:100 +
1:100 +
1:100 -
1:100 -
1:1,000 +
1:1,000 +
1:1,000 -
1:1,000 -
1:10,000 +
1:10,000 +
1:10,000 -
1:10,000 -
1:100,000 +
1:100,000 +
1:100,000 -

Fig. 4

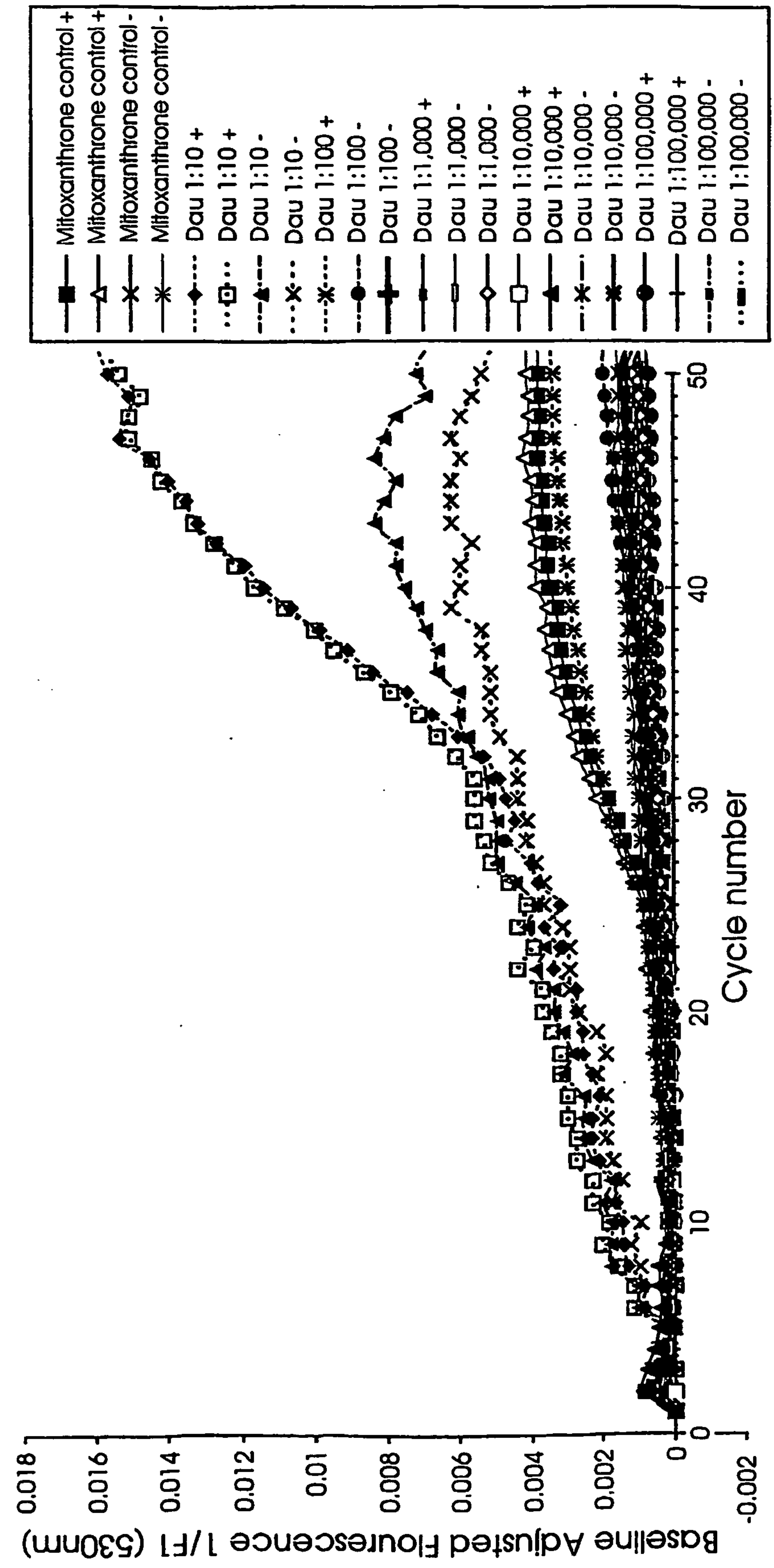
Baseline Adjusted Flouresence 1/F1 (530nm)
0.018
0.016
0.014
0.012
0.01
0.008
0.006
0.004
0.002
0
-0.002
0
10
20
30
40
50
Cycle number
Mitoxanthrone control +
Mitoxanthrone control +
Mitoxanthrone control -
Mitoxanthrone control -
Dau 1:10 +
Dau 1:10 +
Dau 1:10 -
Dau 1:10 -
Dau 1:100 +
Dau 1:100 -
Dau 1:100 -
Dau 1:1,000 +
Dau 1:1,000 -
Dau 1:1,000 -
Dau 1:10,000 +
Dau 1:10,000 +
Dau 1:10,000 -
Dau 1:10,000 -
Dau 1:100,000 +
Dau 1:100,000 +
Dau 1:100,000 -
Dau 1:100,000 -

Fig. 5

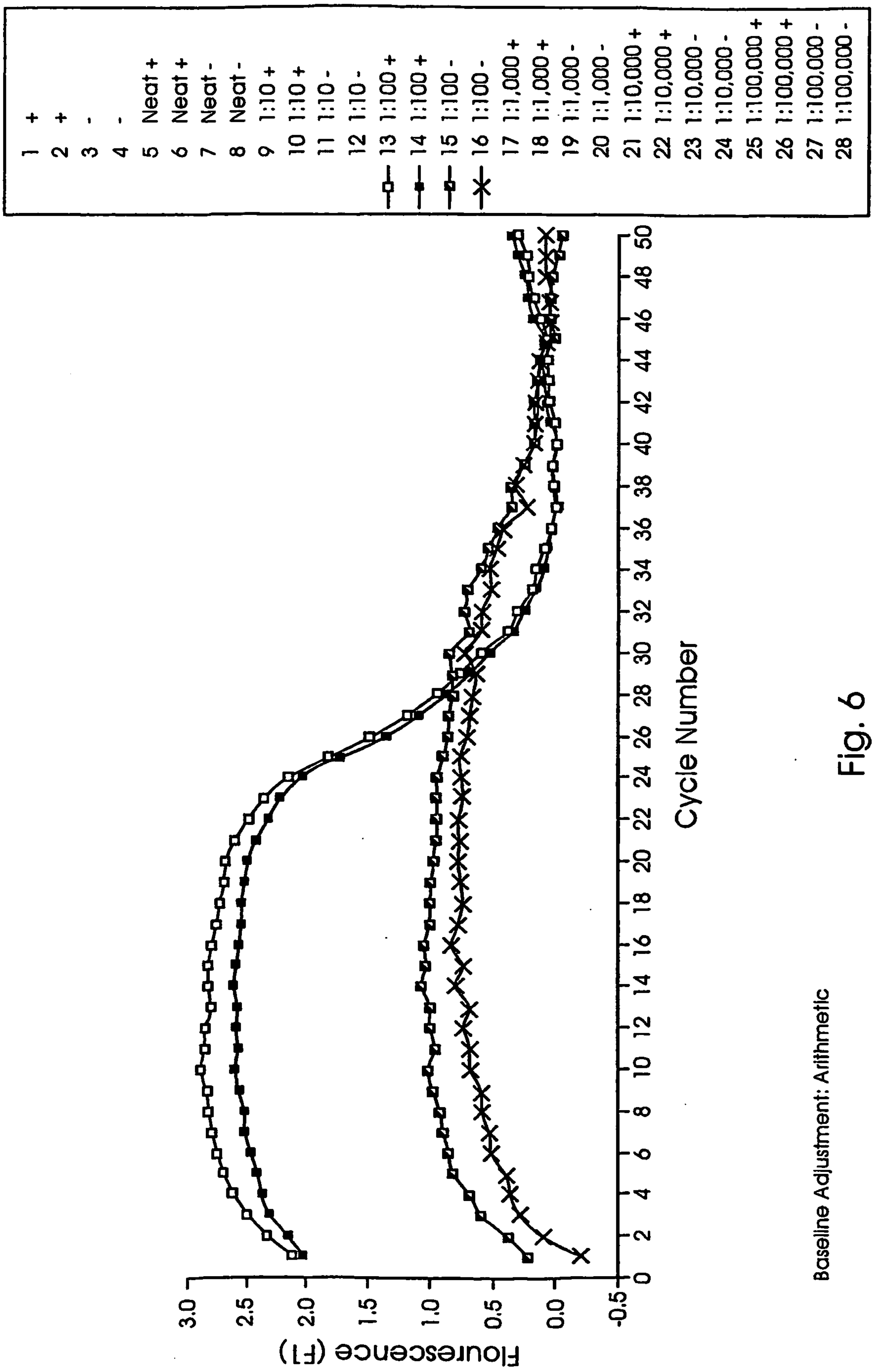
1 +
2 +
3 -
4 -
5 Neat +
6 Neat +
7 Neat -
8 Neat -
9 1:10 +
10 1:10 +
11 1:10 -
12 1:10 -
13 1:100 +
14 1:100 +
15 1:100 -
16 1:100 -
17 1:1,000 +
18 1:1,000 +
19 1:1,000 -
20 1:1,000 -
21 1:10,000 +
22 1:10,000 +
23 1:10,000 -
24 1:10,000 -
25 1:100,000 +
26 1:100,000 +
27 1:100,000 -
28 1:100,000 -
Flourescence (F1)
3.0
2.5
2.0
1.5
1.0
0.5
0.0
-0.5
0 2 4 6 8 10 12 14 16 18 20 22 24 26 28 30 32 34 36 38 40 42 44 46 48 50
Cycle Number
Baseline Adjustment: Arithmetic

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 872562 A
- EP 0699768 A
- WO 9928500 A
- US 4197249 A
- US 3183157 A
- US 4012284 A
- US 3997662 A
- US 513327 A
- US 5132327 A
- EP 0450021 A
- GB 0201861 W